# EUROPEAN PATENT SPECIFICATION

(11) **EP 2 027 833 B1**
(45) Date of publication and mention of the grant of the patent: **02.07.2014**
(21) Application number: 08252772.2
(22) Date of filing: 21.08.2008
(51) Int. Cl.: A61F 2/36

(54) **Trial prosthetic neck component**
Versuchsprothesenhalskomponente
Composant de prothèse de cou d'essai

(30) Priority: 22.08.2007 GB 0716403
(43) Date of publication of application: 25.02.2009
(73) Proprietor: Stryker Ireland Limited, Carrigtwohill, Cork (IE)
(72) Inventor: Le Bon, Franck, 14210 Evrecy (FR); Collet, Pascal, 14320 Saint Martin de Fontenay (FR)
(74) Representative: Bridge-Butler, Jeremy

(56) References cited:
- EP-A- 1 566 155
- WO-A-00/41653
- WO-A-96/00539
- DE-U1- 9 418 963
- US-A1- 2003 233 100
- US-A1- 2004 064 186

## Description

This invention relates to a trial prosthetic neck component which is adapted for use with a trial femoral stem or broach or an implanted femoral stem.

The trial prosthetic neck component, according to the invention, is intended for use in a modular operative technique before fitting a femoral implant which employs a modular prosthetic neck component the distal end of which is attached to a femoral stem and the proximal end of which carries or is attached to a bearing head in the shape of a part-spherical ball. The use of the modular neck component enables the surgeon to provide any required offset to suit the joint at the time of implantation.

WO 96/00539 discloses a trial prosthetic neck component which has removable connection means to a femoral stem of a femoral trial prosthesis and the proximal and distal components can be combined to form a femoral trial prosthesis that corresponds to an available prosthetic femoral implant. This construction does not include means for connecting the neck component to a broach which can be used to replace the femoral stem and there is no provision for indicating the length and any valgus, varus, anteverted, retroverted offset.

According to the present invention a modular trial prosthetic neck component a modular neck the proximal end of which has connection means for removably connecting a bearing head and the distal end of which has a connecting means for removably connecting it by a first form of connection to a trial medullary stem or the actual femoral stem which is to be used in the prosthesis characterised in that the distal end of said modular neck is also provided with connection means for removably connecting it by a second form of connection to a broach and in which said proximal end is provided with indicea to indicate its length and any valgus, varus, anteverted, retroverted offset.

Thus, a number of trial neck components, as set out above, can be provided and the operating surgeon can select an appropriate trial neck having the necessary offset and length to suit the patient. He can assess the neck component when fitted or carrying the bearing head and by selecting the appropriate component achieve the desired result for a good fit. The indicea provided on the end of the component enables the surgeon to then select the appropriate modular neck component from a supply and will enable the surgeon to build up the implant as required.

In a preferred embodiment the indicea indicating offset comprises a line extending in the direction of the offset.

With this arrangement the indicea which indicates the length of the component can be provided by a coloured dot the longer the length the greater the diameter of the dot.

Each line indicating the offset preferably extends outwardly from the length indicating dot provided at a central point on the proximal end of the neck component. The line thus points in the direction of the offset and extends away from the length indicating dot.

The connection means preferably includes a removable connector which when located on the modular neck can be used to connect it to a trial stem or actual femoral stem by a first form of connection and which can be removed from the modular neck to allow the neck to be connected to a broach by a second form of connection.

The second form of connecting means to the broach can include an angularly located projecting abutment and a socket.

With this arrangement the projecting abutment and socket on the neck are dimensioned to engage and fit a socket and plug on said broach and the proximal end of the connector has a central plug and an angularly located socket to engage the abutment and the socket on the neck component, and the distal end of said connector has a spade connector to engage and located in the trial stem or implanted femoral stem.

With this arrangement the proximal end of the connector may include a plug and an angularly located socket and the distal end of the neck component can have a socket to receive said plug and a projecting abutment to engage the angularly located socket. This arrangement ensures that the connector is fixed against relative angular rotation to the stem when in position.

In order to provide a connection which can be a tight push fit the connector can be made of a synthetic plastics material.

The trial prosthetic neck component, according to the invention, can be provided in combination with a number of other similar trial neck components which have different lengths and/or offsets and which can be selectively connected to a trial medullary stem or broach or an implanted femoral stem and a bearing head to provide a modular construction of trial femoral implants of different lengths and offsets so that a surgeon can achieve the desired length and offset of either a trial implant provided by a trial stem or a broach or by fitting the trial neck component to a femoral stem which has already been implanted.

The trial prosthetic neck component can therefore be provided in combination with seventeen similar trial neck components, nine of the components having long necks, three of said long neck components having a valgus offset and one of which also has an anteverted offset, another of which also has a retroverted offset and a third of which has no anteverted or retroverted offset; another three of said long neck components having a varus offset one of which also has an anteverted offset another of which also has a retroverted offset and a third of which has no anteverted or retroverted offset; and the remaining three of said long neck components having no valgus or varus offset, one of said three having an anteverted offset, another having a retroverted offset and a third of which has no anteverted or retroverted offset; the remaining nine neck components having short necks and replicating the various offsets of the nine long neck components.

With this arrangement the operating surgeon is able to choose a trial neck component which is of long or short neck length and which has offsets in all the available directions. The indicea on the proximal end of the component which he/she has selected enables him/her to then take the appropriate modular component which is to be secured to the stem.

In a preferred arrangement each of the nine long neck trial components carries a coloured dot to indicate the particular offset, each two components have offsets which are the reciprocal of each other and one of which has a valgus effect and the other a varus offset having the same colour, and the components which do not have a valgus or varus offset having the same colour; and the nine neck components having short necks and replicating the various offsets of the nine long neck components having a similar colour coding.

The coloured dot which indicates the length of the component can also carry the colour coding which indicates the offset.

Suitable colours are brown, green, blue, yellow and grey.

This arrangement enables the use of only ten modular necks for use in the implanted stem. Because they can be constructed so that in each of the constructions they can be used in the reciprocal directions merely by rotating them through 180°, thus a neck which has a varus anteverted offset can also be used by rotating it through 180° as a neck valgus and retroverted offset.

The modular necks which are to be inserted in the fixed stem also, preferably, carry the colour coding of the test necks so that if, for example, the colour of the neck which had varus and anteverted offset is yellow and can be used with an implanted stem could be used by the surgeon but he could also use the same yellow neck if the test neck indicated that an offset of varus retroverted was required merely by rotating the modular stem to the appropriate direction. Modular necks could also carry the direction indicators as used by the test necks to enable this to be done.

The invention also includes a trial prosthetic neck component as set forth above in combination with seventeen similar trial neck components which have the colour coding and offset indicea form part of a kit of parts which also includes ten modular necks of different offsets which can be used when the required offset has been determined by the trial offset necks and one or more modular stems which were used to replace the trial broach or stem, together with one or more trial broaches or stems.

The kit of parts could also include one or more bearing heads attachable to the appropriate modular neck or the modular necks could carry integral bearing heads.

The spade is provided on the distal end of the connector. A similar spade-shape connector is provided on the modular necks but in this case are a tight or interference fit whereas the spade on the connector which is preferably made of a synthetic plastics material is a tight or push fit into the slot.

The invention can be performed in various ways and one embodiment will now be described by way of example and with reference to the accompanying drawings in which :-
Figure 1 is a diagrammatic isometric view showing the proximal end of a femur the head of which has been resected to receive an implant having a stem located within the femur and projecting neck to which a bearing, in the form of a hemispherical ball can be fitted;
Figure 2 is an isometric view of a modular neck similar to that shown in Figure 1 which can be located in an implanted stem;
Figure 3 is a diagrammatic isometric view from one side of a trial prosthetic neck component, according to the invention;
Figure 4 is an isometric view from the front of eighteen alternative trial neck components according to the invention;
Figure 5 is a diagrammatic view of the proximal ends of the components shown in Figure 4;
Figure 6 shows left and right handed prosthetic stems which can be used with the present invention; and,
Figure 7 is a diagrammatic view of the proximal end of a broach with which the invention can be used.

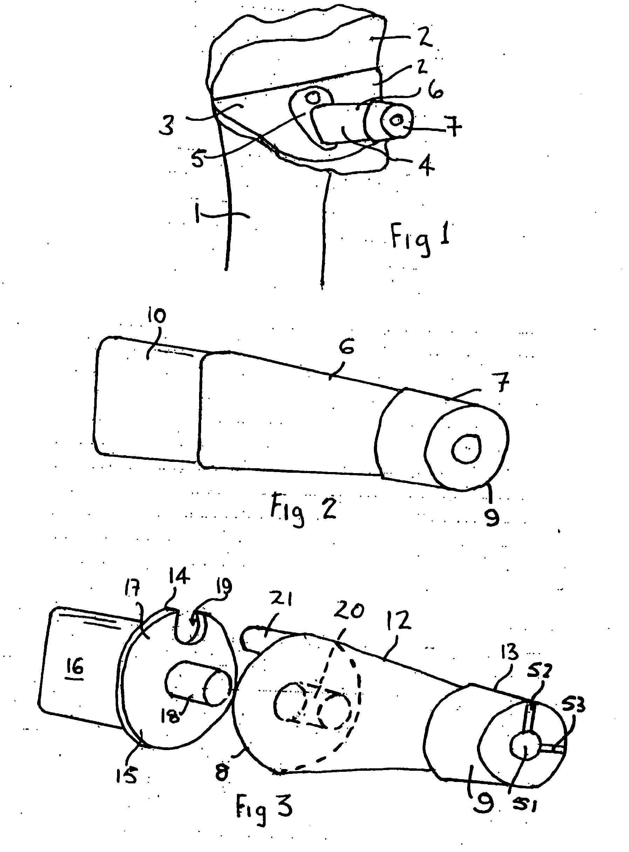

Figure 1 is a diagrammatic illustration of the proximal end of a femur 1 which is resected at 2 to provide a flat planar surface 3 in which a femoral prosthesis 4 has been implanted. The prosthesis 4 is of modular construction and comprises a stem 5 located in the femur, a modular neck 6, the distal end 8 of which is secured to the stem 5 and the proximal end 9 of which is provided with a conical boss 7 which has a Morse taper and on which a spherical or part-spherical bearing head can be mounted.

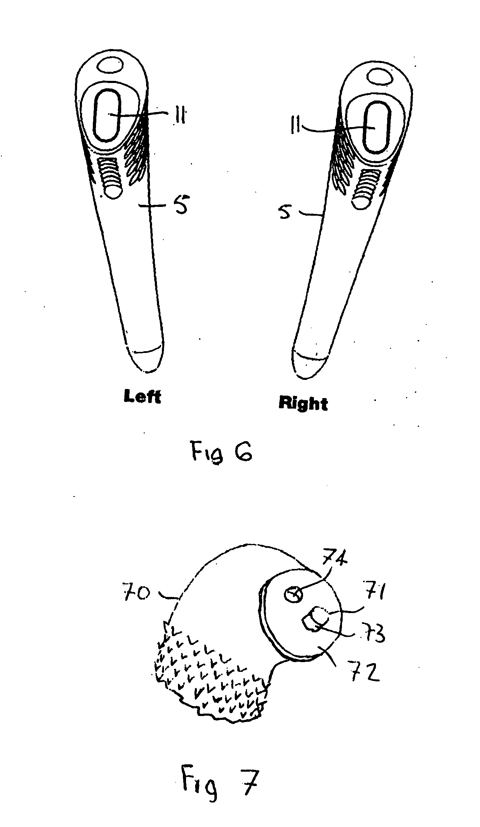

As will be seen from Figure 2 the distal end of the modular neck 6 is provided with an engagement spade 10 which is shaped to locate in an elongated slot 11 which is most clearly shown in Figure 6. The engagement spade 10 is dimensioned to be pushed or driven into the elongate slot by providing a tight or interference fit. Spade 10 may have curved ends connected by straight portions.

As will be seen from Figure 6 the modular femoral stem 5 can be made in left or right-handed versions.

Figure 7 shows the proximal end of a broach 70 which can be used with the present invention and which has connection means 71 which include a flange 72 which has a central plug 73 and an angularly located socket 74 to receive a handle or other equipment.

Before fitting an implant of the type shown in Figure 1 it is usual to provide a trial component to enable the surgeon to assess the necessary dimensions and consider whether there should be any degree of offset of the modular neck in relation to the stem.

The present invention, therefore, provides a trial prosthetic neck component which can be used with a trial femoral stem or broach, if suitable, or with an already implanted modular femoral stem. The trial prosthetic neck component is shown in Figure 3 and comprises a shaped neck 12, the proximal end 9 of which carries a tapered engagement boss 13 for a spherical bearing head (not shown) and the distal end 8 of which has connection means 14 for removably connecting it to the proximal end of the trial femoral stem or broach or the implanted stem. The trial stem can be basically similar to the stem 5 of the implanted prosthesis or it can be in the form of a broach as shown in Figure 7 which has been used to define the opening in the bone. The broach is left in position and used as a trial stem. If a trial stem is used it is provided with an elongated slot 11 similar to that shown in Figure 6 to receive the trial neck. The connection means 14 are therefore provided with a connector 15 which has a distal spade-shaped end 16 similar to the shape-shaped end 10 of the modular neck 6. The spade end 16 extends from a flange 17 which has a central plug 18 and an angularly located socket 19. The distal end 8 of the neck component 12 has a socket 20 to receive the plug 18 and a projecting abutment 21 to engage the angularly located socket 19. The connector 15 can be made from a synthetic plastics material so that when it is assembled to the trial neck the plug 18 is a tight push fit into the socket 19 and the abutment 21 is a tight fit in the socket 19 so that the connector is firmly held in position. If the broach 70 is used for trials then the socket 20 and projecting abutment 21 can be used to engage the central plug 73 and socket 74, in the broach when connector 15has been removed.

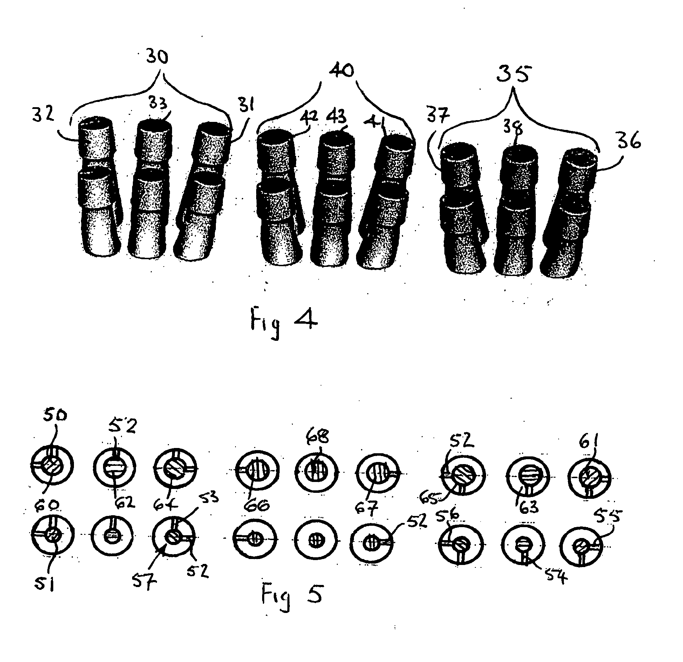

A number of trial necks are provided in different lengths and offsets so that the surgeon can select the trial neck which is appropriate for the patient. Figure 4 illustrates eighteen modular trial necks of the kind shown in Figure 3. In Figure 4 the rear row of components all have long necks. The left hand group of three of these long-necked components and indicated by reference numeral 30 have valgus offset, one of which indicated by reference numeral 31 also has an anteverted offset and that indicated by reference numeral 32 has a retroverted offset and that indicated by reference numeral 33 has no antevrted or retroverted offset.

In the next group of neck components, indicated by reference numeral 35, in varus offset one of which indicated by reference numeral 36 has an anteverted offset, another, indicated by reference numeral 37, has a retroverted offset and the third, indicated by reference numeral 38

The remaining three of said long neck components and indicated by reference numeral 40 have no valgus or varus offset, one of the said three, indicated by reference numeral 41, has an anteverted offsest, another indicated by reference numeral 42, has a retroverted offset and the third, indicated by reference numeral 43, has no anteverted or retroverted offset .

The trial neck components shown in the front row in Figure 4 are similar to those shown in the back row and indicated by reference numerals 30 to 43 but all the front row components have a short neck.

The two lengths of neck and various offsets enable the surgeon to assemble the required length and offset but once he has selected the trial neck he requires he has to identify that neck with the modular necks which are to be used in the implant, and in order to assist the surgeon the proximal end faces of the trial necks carry indicea to indicate its length and any valgus, varus, anteverted, reverted offset.

Figure 5 shows how the indicea is applied. The indicea indicating offset comprises a line extending in the direction of the offset. The indicea which indicates the length of the component is provided by a coloured dot, the longer the length of the component the greater the diameter of the dot.

Each line indicating the offset extends outwardly from the length indicated dot which is provided as a central point on the proximal end of the neck component.

Inspection of Figure 5 will show that the upper row of end surfaces all carry a large coloured dot 50. All the lower row of end faces carry a small dot 51 to indicate that they are of the shorter length.

As will be seen the indicating lines 52 can extend in various directions according to their requirement, for example, a large central dot 50 indicates a long neck whereas a smaller dot (indicated by reference numeral 51) will indicate a short neck. No line associated to the dot indicates that the neck is neither varus nor valgus and neither anteverted nor retroverted. A vertical line, such as indicated by reference numeral 53, would indicate a valgus neck whereas a vertical line, as indicated by reference numeral 54, would indicate a varus neck. A horizontal line, such as indicated by reference numeral 55 indicates an anteverted neck and a horizontal line, as indicated by reference numeral 56, indicates a retroverted neck.

As a further example, a horizontal and vertical line such as shown at reference numeral 57 indicates a valgus neck which is anteverted.

Ten modular necks will be available to the surgeon, five of long length and five of short. As the spade stem 10 of the modular necks can be rotated through 180° only five of said necks of each length would be necessary. In order to identify the necks the coloured dots and lines are of different colours. As shown in Figure 5 those necks which have offsets which are the reciprocal of each other carry the same colour. Thus, neck 60 with a large dot 50 carries the same colour (yellow) as the head 61 which is its reciprocal when rotated through 180°. Head 62 is the reciprocal of head 63 and thus carries the same colour (green). Head 64 is the reciprocal of head 65 and carries the same colour (blue), head 66 is the reciprocal of head 67 and again carries the same colour (brown). The remaining head which does not have any opposite (head 68) is colour grey. The same colour scheme is also applied to the short length necks.

When carrying out this surgery the surgeon first carries out various tests and the pre-operative planning of such a modular stem system requires three steps,
i). to first determine the centre of rotation of the acetabulum;
ii) to determine the metaphyseal position and sides of the femoral component;
iii) to determine the proper modular neck in terms of leg length and offset.

Other tests are carried out to decide on the choice of femoral component and the final choice of component size will depend on the intra-operative rotational stability of the broach.

The surgeon will first resect the femoral neck until the metaphyseal housing of the implant is prepared. A hollow chisel is preferably used which is adapted to the size of the implant and fixed to a broach handle. A cylinder of cancellous bone is removed from the metaphysis taking care not to damage the calcar of the greater trochanter. The smallest broach, left or right, is then introduced into the final medullary canal. Flexible reamers can be used up to the diameter corresponding to the chosen implant. After checking that they correspond to the side being operated broaches are fixed to the broach handle and they are introduced beginning with the smaller size up the size chosen during pre-op planning. The broach and and/or a trial prosthesis size will determine the size of the permanent implant. If the broach is unstable then a larger broach may be necessary.

A metal trial neck from the broach featuring the CCD angle and neck length chosen during pre-op templating, combined with a standard anteversion can be used first. The CCD angle on a neck length is selected by checking leg length and soft tissue tension and various head lengths can be used to enable fine tuning.

Once the most suitable CCD angle and neck head length have been determined the choice of the neck will be finally obtained by a good internal/external rotational ability in extension and a good stability of the hip in flexion/internal rotation/aduction and in extension external rotation/abduction.

In order to obtain these settings the trial neck components are used until the necessary position and length is found.

The trial neck and its connection are now removed together with the broach or trial stem and the modular stem which is coated with hydroxyapatite is introduced into the bone until it becomes wedged. The wedging can be completed by gentle hammering.

The stem can have a small plastic stem holder at its proximal end to enable the surgeon to handle it without damage.

With the stem now in position further trials can be carried out again using the trial necks. To do so the trial neck which is selected needs to be attached to the connector which would be seated on the stem and the whole trial process will take place again, that is checking the CCD angle and length and checking the version.

With the correct trial neck now established the surgeon can take one of the ten modular heads supplied in the kit, the colour and indicating lines showing which modular head can be used.

Once the implant is selected the insertion in the proper orientation is enabled with reference to the orientation of the colours and lines.

The modular stem is now inserted with its spade end in the elongated opening 11 and hammered into position. The bearing head (not shown) can now be fitted.

The invention provides a test neck which, when used, indicates to the surgeon the precise type of modular neck which can be used to obtain the desired results.

The proximal ends of the trial stems can also carry other information, for example size, numbers and any other indicea which may be of use to the surgeon.

## Claims

1. A modular trial prosthetic neck component having a modular neck (6) the proximal end of which has connection means (7) for removably connecting a bearing head and the distal end of which has a connecting means for removably connecting it by a first form of connection to a trial medullary stem or the actual femoral stem (5) which is to be used in the prosthesis **characterised in that** the distal end (8) of said modular neck (6) is also provided with connection means (14) for removably connecting it by a second form of connection to a broach (70) and in which said proximal end (9) is provided with indicea (50-56) to indicate its length and any valgus, varus, anteverted, retroverted offset.

2. A modular trial prosthetic neck component as claimed in claim 1 **characterised in that** said indicea indicating offset comprises a line (52) extending in the direction of the offset.

3. A modular trial prosthetic neck component as claimed in claim 1 or claim 2 **characterised in that** the indicea which indicates its length is provided by a coloured dot (50, 51) the longer the length the greater the diameter of the dot.

4. A modular trial prosthetic neck component as claimed in claim 3 when dependent on claim 2 **characterised in that** each line (52) indicating the offset extends outwardly from the length indicating circular dot (50, 51) provided at a central point on the proximal end (9) of the component.

5. A modular trial prosthetic neck component as claimed in claims 1 to 4 **characterised in that** the connection means (14) include a removable connector (15) which when located on the modular neck (6) can be used to connect it to a trial stem or actual femoral stem (5) by a first form of connection and which can be removed from the modular neck (6) to allow the neck (6) to be connected to a broach (70) by a second form of connection.

6. A modular trial prosthetic neck component as claimed in any one of the preceding claims **characterised in that** the second form of connecting means to the broach includes an angularly located socket (20) and a projecting abutment (21).

7. A modular trial prosthetic neck component as claimed in claim 5 or claim 6 **characterised in that** the projecting abutment (21) and socket (20) on the neck are dimensioned to engage and fit a socket (74) and plug (73) on said broach (70) and the proximal end of the connector (15) has a central plug (18) and an angularly located socket (19) to engage the abutment (21) and the socket (20) on the neck component, and the distal end of said connector (15) has a spade connector (16) to engage and locate in the trial stem or implanted femoral stem (5).

8. A modular trial prosthetic neck component as claimed in claims 5, 6 or 7 **characterised in that** the connector (15) is made from a synthetic plastics material.

9. A modular trial prosthetic neck component as claimed in claims 1 to 8 **characterised in that** it is provided in combination with a number of other similar neck components (6) which have different lengths and/or offsets and which can be selectively connected to a trial medullary stem or broach (7) or the actual stem (5) and a bearing head to provide a modular construction of trial femoral implants of different lengths and offsets.

10. A modular trial prosthetic neck component as claimed in claim 9 which is provided in combination with seventeen similar neck components (6), nine of the components having long necks, three of said long neck components having a valgus offset one of which also has an anteverted offset, another of which also has a retroverted offset, and the third of which has no anteverted or retroverted offset;
three of said long neck components having a varus offset one of which also has an anteverted offset, another of which also has a retroverted offset and the third of which has no anteverted or retroverted offset, and the remaining three of said long neck components having no varus or valgus offset, one of said three having an anteverted offset, another having a retroverted offset, and a third of which has no anteverted or retroverted offset; the remaining nine neck components having short necks and replicating the various offsets of the nine long neck components.

11. A modular trial prosthetic neck component as claimed in claim 10 **characterised in that** each of the nine long neck trial components carries a coloured dot (50, 51) to indicate the particular offset, each two components have offsets which are the reciprocal of each other and one of which has a valgus offset and the other a varus offset having the same colour, and the three components which do not have a valgus or varus offset having the same colour; and the other components having short necks and which do not have a valgus or varus offset having the same colour; and the nine neck components having short necks and replicating the various offsets of the nine neck components having a similar coding.

12. A modular trial prosthetic neck component as claimed in claim 11 **characterised in that** a coloured dot (50, 51) which indicates the length of the component (6) also carries the coloured coding which indicates the offset.

13. A modular trial prosthetic neck component as claimed in claim 11 or claim 12 **characterised in that** the colours are brown, green, blue, yellow and grey.

14. A modular trial prosthetic neck component as claimed in claims 10, 11 or 12 **characterised in that** the modular necks (6) which are to be inserted in an actual fixed stem (5) also carry the colour coding of the trial necks (6).

15. A modular trial prosthetic neck component as claimed in claim14 **characterised in that** modular necks which are to be inserted in an actual fixed stem (5) also carry the direction indicators as used on the trial necks (6).

16. A modular trial prosthetic neck component as claimed in any one of the preceding claims **characterised by** being in combination with nineteen similar trial neck components (6) which have the colour coding and offset indicea form a kit of parts which also includes ten modular necks of different offsets which can be used when the required offset has been determined by the trial offset necks (6), and one or more modular stems which are used to replace the trial broach or stem together with one or more trial broaches (70) or stems (71).

17. A modular trial prosthetic neck component as claimed in claim16 **characterised in that** the kit of parts includes one or more bearing heads attachable to the appropriate modular neck.

18. A modular trial prosthetic neck component as claimed in claim16 **characterised in that** the kit of parts includes one or more modular necks carrying integral bearing heads.

## Patentansprüche

1. Modulare prothetische Probe-Halskomponente mit einem modularen Hals (6), dessen proximales Ende Verbindungsmittel (7) zum abnehmbaren Verbinden eines Lagerkopfs aufweist und dessen distales Ende ein Verbindungsmittel aufweist, um es durch eine erste Form von Verbindung abnehmbar mit einem medullären Probe-Schaft oder dem tatsächlichen femoralen Schaft (5), der in der Prothese benutzt werden soll, zu verbinden, **dadurch gekennzeichnet, dass** das distale Ende (8) des modularen Halses (6) auch mit Verbindungsmitteln (14) versehen ist, um es durch eine zweite Form von Verbindung abnehmbar mit einem Räuminstrument (70) zu verbinden, und wobei das proximale Ende (9) mit Markierungen (50-56) versehen ist, um seine Länge und jeglichen Valgus-, Varus-, Anteversions-, Retroversions-Offset anzuzeigen.

2. Modulare prothetische Probe-Halskomponente nach Anspruch 1, **dadurch gekennzeichnet, dass** die Offset anzeigende Markierung eine Linie (52) umfasst, die sich in der Richtung des Offset erstreckt.

3. Modulare prothetische Probe-Halskomponente nach Anspruch 1 oder Anspruch 2, **dadurch gekennzeichnet, dass** die Markierung, die seine Länge anzeigt, durch einen farbigen Punkt (50, 51) bereitgestellt ist, wobei der Durchmesser des Punktes umso größer ist, je größer die Länge ist.

4. Modulare prothetische Probe-Halskomponente nach Anspruch 3 rückbezogen auf Anspruch 2, **dadurch gekennzeichnet, dass** sich jede den Offset anzeigende Linie (52) von dem an einer zentralen Stelle auf dem proximalen Ende (9) der Komponente vorgesehenen, die Länge anzeigenden kreisförmigen Punkt (50, 51) aus auswärts erstreckt.

5. Modulare prothetische Probe-Halskomponente nach den Ansprüchen 1 bis 4, **dadurch gekennzeichnet, dass** die Verbindungsmittel (14) einen abnehmbaren Verbinder (15) einschließen, der, wenn er auf dem modularen Hals (6) positioniert ist, benutzt werden kann, um ihn durch eine erste Form von Verbindung mit einem Probe-Schaft oder tatsächlichen femoralen Schaft (5) zu verbinden, und der vom modularen Hals (6) abgenommen werden kann, um es zu gestatten, den Hals (6) durch eine zweite Form von Verbindung mit einem Räuminstrument (70) zu verbinden.

6. Modulare prothetische Probe-Halskomponente nach einem der vorangehenden Ansprüche, **dadurch gekennzeichnet, dass** die zweite Form von Verbindungsmitteln zum Räuminstrument eine unter einem Winkel angeordnete Buchse (20) und ein überstehendes Widerlager (21) einschließt.

7. Modulare prothetische Probe-Halskomponente nach Anspruch 5 oder Anspruch 6, **dadurch gekennzeichnet, dass** das überstehende Widerlager (21) und die Buchse (20) auf dem Hals bemessen sind, um mit einer Buchse (74) und einem Bolzen (73) auf dem Räuminstrument (70) in Eingriff zu treten und zusammenzupassen, und das proximale Ende des Verbinders (15) einen mittigen Bolzen (18) und eine unter einem Winkel angeordnete Buchse (19) aufweist, um mit dem Widerlager (21) und der Buchse (20) auf der Halskomponente (6) in Eingriff zu treten, und das distale Ende des Verbinders (15) einen Spatenverbinder (16) aufweist, um mit dem Probe-Schaft oder dem implantierten femoralen Schaft (5) in Eingriff zu treten und sich darin zu positionieren.

8. Modulare prothetische Probe-Halskomponente nach den Ansprüchen 5, 6 oder 7, **dadurch gekennzeichnet, dass** der Verbinder (15) aus einem Kunststoffmaterial hergestellt ist.

9. Modulare prothetische Probe-Halskomponente nach den Ansprüchen 1 bis 8, **dadurch gekennzeichnet, dass** sie in Kombination mit einer Anzahl von anderen ähnlichen Halskomponenten (6) vorgesehen ist, die verschiedene Längen und/oder Offsets aufweisen und die wahlweise mit einem medullären Probe-Schaft oder Räuminstrument (7) oder dem tatsächlichen Schaft (5) und einem Lagerkopf verbunden werden können, um eine modulare Konstruktion von femoralen Probe-Implantaten mit verschiedenen Längen und Offsets bereitzustellen.

10. Modulare prothetische Probe-Halskomponente nach Anspruch 9, die in Kombination mit siebzehn ähnlichen Halskomponenten (6) vorgesehen ist, wobei neun der Komponenten lange Hälse aufweisen, wobei drei der Langhalskomponenten einen Valgus-Offset aufweisen, von denen eine auch einen Anteversions-Offset aufweist, von denen eine andere auch einen Retroversions-Offset aufweist und von denen die dritte keinen Anteversions- oder Retroversions-Offset aufweist; wobei drei der Langhalskomponenten einen Varus-Offset aufweisen, von denen eine auch einen Anteversions-Offset aufweist, von denen eine andere auch einen Retroversions-Offset aufweist und von denen die dritte keinen Anteversions- oder Retroversions-Offset aufweist, und wobei die verbleibenden drei der Langhalskomponenten keinen Varus- oder Valgus-Offset aufweisen, wobei eine der drei einen Anteversions-Offset aufweist, eine andere einen Retroversions-Offset aufweist und von denen eine dritte keinen Anteversions- oder Retroversions-Offset aufweist; wobei die verbleibenden neun Halskomponenten kurze Hälse aufweisen und die verschiedenen Offsets der neun Langhalskomponenten replizieren.

11. Modulare prothetische Probe-Halskomponente nach Anspruch 10, **dadurch gekennzeichnet, dass** jede der neun Probe-Langhalskomponenten einen farbigen Punkt (50, 51) trägt, um den speziellen Offset anzuzeigen, dass jeweils zwei Komponenten Offsets aufweisen, die das Umgekehrte voneinander sind und von denen eine einen Valgus-Offset und die andere einen Varus-Offset mit derselben Farbe aufweist, und die drei Komponenten, die keinen Valgus- oder Varus-Offset aufweisen, dieselbe Farbe besitzen; und die anderen Komponenten, die kurze Hälse aufweisen und die keinen Valgus- oder Varus-Offset aufweisen, dieselbe Farbe besitzen; und die neun Komponenten, die kurze Hälse aufweisen und die verschiedenen Offsets der neun Halskomponenten replizieren, eine ähnliche Codierung besitzen.

12. Modulare prothetische Probe-Halskomponente nach Anspruch 11, **dadurch gekennzeichnet, dass** ein farbiger Punkt (50, 51), der die Länge der Komponente (6) anzeigt, auch die farbige Codierung trägt, die den Offset anzeigt.

13. Modulare prothetische Probe-Halskomponente nach Anspruch 11 oder Anspruch 12, **dadurch gekennzeichnet, dass** die Farben Braun, Grün, Blau, Gelb und Grau sind.

14. Modulare prothetische Probe-Halskomponente nach den Ansprüchen 10, 11 oder 12, **dadurch gekennzeichnet, dass** die modularen Hälse (6), die in einen tatsächlichen befestigten Schaft (5) eingesetzt werden sollen, auch die Farbcodierung der Probe-Hälse (6) tragen.

15. Modulare prothetische Probe-Halskomponente nach Anspruch 14, **dadurch gekennzeichnet, dass** die modularen Hälse (6), die in einen tatsächlichen befestigten Schaft (5) eingesetzt werden sollen, auch die Richtungsanzeigen tragen, wie sie auf den Probe-Hälsen (6) verwendet werden.

16. Modulare prothetische Probe-Halskomponente nach einem der vorangehenden Ansprüche, **dadurch gekennzeichnet, dass** sie in Kombination mit neunzehn ähnlichen Probe-Halskomponenten (6), welche die Farbcodierung und Offset-Markierungen aufweisen, einen Satz von Teilen bildet, der auch zehn modulare Hälse mit verschiedenen Offsets einschließt, die benutzt werden können, wenn der erforderliche Offset mittels der Probe-Offset-Hälse (6) bestimmt worden ist, sowie einen oder mehrere modulare Schäfte, die benutzt werden, um das Probe-Räuminstrument oder den Probe-Schaft zu ersetzen, zusammen mit einem oder mehreren Probe-Räuminstrumenten (70) oder Schäften (71).

17. Modulare prothetische Probe-Halskomponente nach Anspruch 16, **dadurch gekennzeichnet, dass** der Satz von Teilen einen oder mehrere Lagerköpfe einschließt, die sich an dem passenden modularen Hals anbringen lassen.

18. Modulare prothetische Probe-Halskomponente nach Anspruch 16, **dadurch gekennzeichnet, dass** der Satz von Teilen einen oder mehrere modulare Hälse einschließt, die integrale Lagerköpfe tragen.

## Revendications

1. Composant de col modulaire prothétique d'essai ayant un col modulaire (6), dont l'extrémité proximale a des moyens de raccordement (7) pour raccorder de manière amovible une tête d'appui et dont l'extrémité distale a un moyen de raccordement pour la raccorder de manière amovible par une première forme de raccordement à une tige médullaire d'essai ou la véritable tige fémorale (5) qui doit être utilisée dans la prothèse, **caractérisé en ce que** l'extrémité distale (8) dudit col modulaire (6) est également prévue avec des moyens de raccordement (14) pour la raccorder de manière amovible par une seconde forme de raccordement à une broche (70) et dans lequel ladite extrémité proximale (9) est prévue avec des indices (50 - 56) pour indiquer sa longueur et tout valgus, varus, décalage antéversé, rétroversé.

2. Composant de col modulaire prothétique d'essai selon la revendication 1, **caractérisé en ce que** ledit indice indiquant le décalage comprend une ligne (52) s'étendant dans la direction du décalage.

3. Composant de col modulaire prothétique d'essai selon la revendication 1 ou la revendication 2, **caractérisé en ce que** l'indice qui indique sa longueur, est fourni par un point coloré (50, 51) pour lequel plus la longueur est importante plus le diamètre du point est important.

4. Composant de col modulaire prothétique d'essai selon la revendication 3, lorsqu'elle dépend de la revendication 2, **caractérisé en ce que** chaque ligne (52) indiquant le décalage s'étend vers l'extérieur à partir du point circulaire indiquant la longueur (50, 51) prévu au niveau d'un point central sur l'extrémité proximale (9) du composant.

5. Composant de col modulaire prothétique d'essai selon les revendications 1 à 4, **caractérisé en ce que** les moyens de raccordement (14) comprennent un connecteur amovible (15) qui, lorsqu'il est situé sur le col modulaire (6), peut être utilisé pour le raccorder à une tige d'essai ou la véritable tige fémorale (5) par une première forme de raccordement et qui peut être retiré du col modulaire (6) pour permettre au col (6) d'être raccordé à une broche (70) par une seconde forme de raccordement.

6. Composant de col modulaire prothétique d'essai selon l'une quelconque des revendications précédentes, **caractérisé en ce que** la seconde forme pour raccorder les moyens à la broche comprend un élément femelle (20) positionné de manière angulaire et une butée en saillie (21).

7. Composant de col modulaire prothétique d'essai selon la revendication 5 ou la revendication 6, **caractérisé en ce que** la saillie de butée (21) et l'élément femelle (20) sur le col sont dimensionnés pour mettre en prise et monter un élément femelle (74) et un élément mâle (73) sur ladite broche (70) et l'extrémité proximale du connecteur (15) a un élément mâle central (18) et un élément femelle (19) positionné de manière angulaire pour mettre en prise la butée (21) et l'élément femelle (20) sur le composant de col, et l'extrémité distale dudit connecteur (15) a un connecteur à broches (16) pour se mettre en prise avec et se positionner dans la tige d'essai ou la tige fémorale (5) implantée.

8. Composant de col modulaire prothétique d'essai selon les revendications 5, 6 ou 7, **caractérisé en ce que** le connecteur (15) est réalisé à partir d'une matière plastique synthétique.

9. Composant de col modulaire prothétique d'essai selon les revendications 1 à 8, **caractérisé en ce qu'**il est prévu en combinaison avec un certain nombre d'autres composants de col (6) similaires qui ont des longueurs différentes et/ou des décalages différents et qui peuvent être sélectivement raccordés à une tige médullaire d'essai ou broche (7) ou la véritable tige (5) et à une tête d'appui pour fournir une construction modulaire d'implants fémoraux d'essai de différentes longueurs et différents décalages.

10. Composant de col modulaire prothétique d'essai selon la revendication 9, qui est proposé en combinaison avec dix-sept composants de col (6) similaires, neuf composants ayant des cols longs, trois desdits composants de col long présentant un valgus, dont l'un a également un décalage antéversé, dont un autre a également un décalage rétroversé, et dont le troisième n'a pas de décalage antéversé ni rétroversé; trois desdits composants de col long présentant un varus, dont l'un a également un décalage antéversé, dont un autre a également un décalage rétroversé et dont le troisième n'a pas de décalage antéversé ni rétroversé, et les trois composants de col long restants ne présentant pas de varus ni de valgus, l'un des trois ayant un décalage antéversé, un autre ayant un décalage rétroversé, et dont un troisième n'a pas de décalage antéversé ni rétroversé; les neuf composants de col restants ayant des cols courts et reproduisant les différents décalages des neuf composants de col long.

11. Composant de col modulaire prothétique d'essai selon la revendication 10, **caractérisé en ce que** chacun des neuf composants d'essai de col long porte un point coloré (50, 51) pour indiquer le décalage particulier, chacun des deux composants ont des décalages qui sont la réciproque l'un de l'autre et dont l'un présente un valgus et l'autre un varus ayant la même couleur, et les trois composants qui ne présentent pas de valgus ni de varus ayant la même couleur; et les autres composants ayant des cols courts et qui ne présentent pas de valgus ni de varus ayant la même couleur; et les neuf composants de col ayant des cols courts et reproduisant les différents décalages des neuf composants de col ayant un code similaire.

12. Composant de col modulaire prothétique d'essai selon la revendication 11, **caractérisé en ce qu'**un point coloré (50, 51) qui indique la longueur du composant (6) porte également le code coloré qui indique le décalage.

13. Composant de col modulaire prothétique d'essai selon la revendication 11 ou la revendication 12, **caractérisé en ce que** les couleurs sont le marron, le vert, le bleu, le jaune et le gris.

14. Composant de col modulaire prothétique d'essai selon les revendications 10, 11 ou 12, **caractérisé en ce que** les cols modulaires (6) qui doivent être insérés dans une véritable tige fixe (5) portent également le code de couleur des cols d'essai (6).

15. Composant de col modulaire prothétique d'essai selon la revendication 14, **caractérisé en ce que** les cols modulaires qui doivent être insérés dans une véritable tige fixe (5) portent également des indicateurs de direction, tels qu'utilisés sur les cols d'essai (6).

16. Composant de col modulaire prothétique d'essai selon l'une quelconque des revendications précédentes, **caractérisé en ce que**, en étant en combinaison avec dix-neuf composants de col d'essai (6) similaires qui ont le code de couleur et des indices de décalage, forment un kit de pièces qui comprend également dix cols modulaires de différents décalages qui peuvent être utilisés lorsque le décalage requis a été déterminé par les cols à décalage d'essai (6), et une ou plusieurs tiges modulaires qui sont utilisées pour remplacer la broche ou tige d'essai conjointement avec une ou plusieurs broches (70) ou tiges (71) d'essai.

17. Composant de col modulaire prothétique d'essai selon la revendication 16, **caractérisé en ce que** le kit de pièces comprend une ou plusieurs têtes d'appui pouvant être fixées au col modulaire approprié.

18. Composant de col modulaire prothétique d'essai selon la revendication 16, **caractérisé en ce que** le kit de pièces comprend un ou plusieurs cols modulaires portant des têtes d'appui solidaires.
